# EUROPEAN PATENT APPLICATION

(11) **EP 2 778 213 A1**
(43) Date of publication of application: **17.09.2014**
(21) Application number: 13159286.7
(22) Date of filing: 14.03.2013
(51) Int. Cl.: C10G 1/00, C12P 7/10, C10L 1/02, C13K 1/02

(54) **Systems and methods for treating biomass**

(71) Applicant: Shell Internationale Research Maatschappij B.V., 2596 HR The Hague (NL)
(72) Inventor: van der Heide, Evert, 1031 HW Amsterdam (NL); MacKay, Munro, 1031 HW Amsterdam (NL)
(74) Representative: Matthezing, Robert Maarten

(57) **Abstract**

Embodiments of the present invention provide a system that uses a reversible acid to treat a biomass in a hydrolysis reaction, where the acid is revertible to components that can be recovered, reused, and/or recycled. In certain embodiments, such components of the reversible acid are neutral. In one embodiment, the acid is recovered in the presence of biomass. In another embodiment, the components of the reversible acid is recycled in the same reactor vessel where the pretreatment occurs. In yet another embodiment, the recycling is done in different reactor vessels. By reusing the reversible acid in the same reactor system, embodiments of the invention eliminates or minimizes subsequent processing steps of the components, such as removal from the pretreatment reactor, recovery, and recycling.

## Description

### Field of the Invention

Embodiments of the invention relate to systems for treating biomass, and more particularly to systems for pretreating biomass with an acid to obtain compounds for use in biofuel or other high value products and recovery of at least a portion of the acid for subsequent uses.

### Background

This section is intended to introduce various aspects of the art, which may be associated with exemplary embodiments of the present invention. This discussion is believed to assist in providing a framework to facilitate a better understanding of particular aspects of the present invention. Accordingly, it should be understood that this section should be read in this light, and not necessarily as admissions of any prior art.

Lignocellulosic biomass is viewed as an abundant renewable resource for fuels and chemicals due to the presence of sugars in the cell walls of plants. More than 50% of the organic carbon on the earth's surface is contained in plants. This lignocellulosic biomass is comprised of hemicelluloses, cellulose and smaller portions of lignin and protein. Cellulose is a polymer comprised mostly of condensation polymerized glucose and hemicellulose is a precursor to pentose sugars, mostly xylose. These sugars can easily be converted into fuels and valuable components, provided they can be liberated from the cell walls and polymers that contain them. However, plant cell walls have evolved considerable resistance to microbial, mechanical or chemical breakdown to yield component sugars. A number of approaches to overcome this recalcitrance have been performed and the breakdown of these polymers into sugars, saccharification, has a long history.

The original approaches dating back to the early 19th century involve complete chemical hydrolysis using concentrated mineral acids such as hydrochloric acid, nitric, or sulfuric acid. Numerous improvements to these processes have been made earning higher sugar yields from the biomass feedstock. These higher acid concentration approaches provide higher yields of sugars, but due to economic and environmental reasons the acids must be recovered. The primary obstacle to practicing this form of saccharification has been the challenges associated with recovery of the acid (M. Galbe and G. Zacchi, A review of the production of ethanol from softwood, Appl. Microbiol. Biotechnol. 59 (2002), pp. 618-628). Recent efforts toward separating sulfuric acid and sugars using ion resin separation or hydrochloric acid and sugars via amine extraction and subsequent thermal regeneration of the acid have been described in US Pat. No. 5,820,687 and WO2010026572. Both approaches are cumbersome and expensive.

Dilute acid processes have also been attempted to perform chemical saccharification and one such example is the Scholler - Tornesch Process. However usage of dilute acid requires higher temperatures and this usually results in low yields of the desired sugars due to thermal degradation of the monosaccharides. Numerous approaches of this type have been made in the past and all have failed to meet economic hurdles. See Lim Koon Ong, Conversion of lignocellulosic biomass to fuel ethanol- A brief review, The Planter, Vol. 80, No. 941, August 2004 and Cell Wall Saccharification, Ralf Möller, Outputs from the EPOBIO project, 2006; Published by CPL Press, Tall Gables, The Sydings, Speen, Newbury, Berks RG14 1RZ, UK.

The saccharification of the cellulose enzymatically holds promise of greater yields of sugars under milder conditions and is therefore considered by many to be more economically attractive. The recalcitrance of the raw biomass to enzymatic hydrolysis necessitates a pretreatment to enhance the susceptibility of the cellulose to hydrolytic enzymes. A number of pretreatment methods, such as described in Nathan Mosier, Charles Wyman, Bruce Dale, Richard Elander, Y. Y. Lee, Mark Holtzapple, Michael Ladisch 'Features of promising technologies for pretreatment of lignocellulosic biomass" Bioresource Technology 96 (2005) pp.673-686, have been developed to alter the structural and chemical composition of biomass to improve enzymatic conversion. Such methods include treatment with dilute acid steam explosion described in US Pat, No. 4,461,648, hydrothermal pretreatment without the addition of chemicals described in WO 2007/009463 A2, ammonia freeze explosion described in AFEX; Holtzapple, M. T., Jun, J., Ashok, G., Patibandla, S.L., Dale, B.E., 1991, The ammonia freeze explosion (AFEX) process-a practical lignocellulose pretreatment, Applied Biochemistry and Biotechnology 28/29, pp. 59-74, and organosolve extraction described in US Pat. No 4,409,032. Despite this, pretreatment has been cited as the most expensive process in biomass-to-fuels conversion ("Methods for Pretreatment of Lignocellulosic Biomass for Efficient Hydrolysis and Biofuel Production" Ind. Eng. Chem. Res., 2009, 48(8), 3713-3729.)

One pretreatment that has been extensively explored is a high temperature, dilute-sulfuric acid (H₂SO₄) process, which effectively hydrolyzes the hemicellulosic portion of the biomass to soluble sugars and exposes the cellulose so that enzymatic Saccharification is successful. The parameters which can be employed to control the conditions of the pretreatment are time, temperature, and acid loading. These are often combined in a mathematical equation termed the combined severity factor. In general, the higher the acid loading employed, the lower the temperature that can be employed; this comes at a cost of acid and its recycle. Conversely, the lower the temperature, the longer the pretreatment process takes; this comes at the cost of volumetric productivity. It is desirable to lower the temperature because pentose sugars readily decompose to form furfurals and other species which represents a yield loss and these compounds are poisons to downstream fermentation. However the use of the higher concentrations of acid required to lower the pretreatment temperatures below that where furfural formation becomes facile (B. P. Lavarack, G. J. Griffin, D. Rodman "The acid hydrolysis of sugarcane bagasse hemicelluloses to product xylose, arabinose, glucose and other products." Biomass and Bioenergy 23 (2002) pp.367-380) once again requires the recovery of the strong acid. If dilute acid streams and higher temperatures are employed the pretreatment reaction produces increased amounts of furfural and the acid passing downstream must be neutralized resulting in inorganic salts which complicates downstream processing and requires more expensive waste water treatment systems.

### Summary

Embodiments of the present invention may address the above challenges by providing a system that uses a reversible acid that is revertible to components, which can be recovered, reused, and/or recycled. In certain embodiments, the components of the reversible acid are neutral. In one embodiment, at least some of the components of the reversible acid are capable of existing in vapor phase. Embodiments of the invention provide benefits of both the concentrated acid hydrolysis methods and the dilute acid biomass pretreatment. In a preferred embodiment, the components of the reversible acid are recycled in the same reactor where the pretreatment occurs. Such a preferred embodiment avoids subsequent processing steps of the components, such as removal from the pretreatment reactor, recovery, and recycling.

In a particular embodiment of the present invention, there is provided a system comprising a first reaction zone having a first set of reaction conditions suitable to drive formation of a reversible acid from one or more components in a vapor phase of the reactor system; a second reaction zone having a second set of reaction conditions suitable to drive separation of the reversible acid to its components; a biomass input for introducing a biomass into the first reaction zone; and a biomass output for discharging the biomass from the second reaction zone; wherein the reactor system is adapted to move the biomass from the first reaction zone to the second reaction zone; and wherein the reactor system is adapted to move the components of the reversible acid from the second reaction zone to the first reaction zone for formation of at least a portion of the reversible acid.

In another embodiment, there is provided a method comprising (a) providing a biomass containing polysaccharides to a first reaction zone having a first set of reaction conditions suitable to drive formation of a reversible acid from one or more components; (b) treating the biomass by allowing the biomass to contact the reversible acid; (c) moving the biomass in contact with the reversible acid from the first reaction zone to a second reaction zone having a second set of reaction conditions suitable to drive separation of the reversible acid to its components; and (d) moving the acid components from the second reaction zone to the first reaction zone for formation of at least a portion of the reversible acid.

In yet another embodiment, there is provided a method comprising: (a) providing a biomass containing polysaccharides to a first reaction zone and forming a reversible acid from one or more components in the first reaction zone; (b) contacting the biomass with the reversible acid in the first reaction zone; (c) moving the biomass in contact with the reversible acid from the first reaction zone to a second reaction zone and separating the reversible acid into one or more components in the second reaction zone; and (d) moving the one or more components from the second reaction zone to the first reaction zone for formation of at least a portion of the reversible acid.

In one embodiment, there is a temperature and/or pressure difference between the reaction zones so that the reversible acid is substantially removed from the biomass in the second reaction zone through vaporization of the reversible acid into its components, at least a portion of which are recombined into the reversible acid at or near the first reaction zone. In one embodiment, the reversible acid is α-hydroxysulfonic acid. In another embodiment, there can be one or multiple reactor vessels. In a further embodiment, there can be recycling of the reversible acid in liquid form. In one embodiment, a compressor is used to facilitate moving of the components of the reversible acid from the second reaction zone to the first reaction zone. In another embodiment, the second reaction zone has a higher temperature than the first reaction zone. In yet another embodiment, there is a gas input in or near the second reaction zone and a gas output in or near the first reaction zone to provide flow of an inert gas during operation to facilitate moving of the reversible acid components from the second reaction zone to the first reaction zone.

Other advantages and features of embodiments of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### Brief Description of the Drawings

These drawings illustrate certain aspects of some of the embodiments of the invention, and should not be used to limit or define the invention.
FIG. 1 is a diagram of an exemplary reactor system according to aspects of the invention comprising one reactor vessel.
FIG. 2 is a diagram of a second exemplary reactor system according to aspects of the invention comprising two reactor vessels.
FIGS. 3A and 3B are diagrams of a third exemplary reactor system according to aspects of the invention comprising three reactor vessels.
FIG. 4 is a diagram of an experimental configuration implemented to demonstrate certain aspects of the invention.
FIG. 5 is a diagram of an exemplary cold trap of condensable materials in the experimental configuration of FIG. 4.
FIG. 6 is a graph of enzymatic hydrolysis results of the experimental samples obtained by experiments conducted using the configuration shown in FIG. 4.

### Detailed Description of Preferred Embodiments

As used herein, the term "biomass" means organic materials produced by plants (e.g., leaves, roots, seeds and stalks). Common sources of biomass include: agricultural wastes (e.g., corn stalks, straw, seed hulls, sugarcane leavings, bagasse, nutshells, and manure from cattle, poultry, and hogs); wood materials (e.g., wood or bark, sawdust, timber slash, and mill scrap); municipal waste (e.g., waste paper and yard clippings); and energy crops (e.g., poplars, willows, switch grass, alfalfa, prairie bluestream, corn, soybean, algae, miscantus, sweet sorghum, energy cane and seaweed). The term "biomass" also refers to the primary building blocks of all the above, including, but not limited to, saccharides, lignins, celluloses, hemicelluloses, and starches. In a preferred embodiment, biomass comprises a lignocellulosic material. Further, in embodiments of the invention, biomass is preferably fed as a solid material.

The term "polysaccharides" refers to polymeric carbohydrate structures, of repeating units (either mono- or di-saccharides) joined together by glycosidic bonds. These structures are often linear, but may contain various degrees of branching. Examples include storage polysaccharides such as starch and glycogen, and structural polysaccharides such as cellulose and chitin. The biomass is preferably preprocessed to suitable particles size. An exemplary preprocessing method may include grinding. Not intending to restrict the scope of the invention, smaller particles of biomass are preferred in certain embodiments for ease of processing. In a preferred embodiment, biomass that is size reduced to facilitate handling (e.g. less than 1.3 cm) is particularly susceptible to saccharification.

Embodiments of the present invention provide a system that uses a reversible acid to treat a biomass in a hydrolysis reaction, where the reversible acid is revertible to components, which can be recovered, reused, and/or recycled. These components of the reversible acid may be referred to as "reversible acid components" or simply as "components." In certain embodiments, such reversible acid components are neutral or non-acidic. Because the reversible acid is revertible to its components under certain conditions, the reversible acid may sometimes be referred to as a "revertible acid." That is, the two terms may be used interchangeably.

In a preferred embodiment, the reversible acid is recovered in the presence of biomass. The components of the reversible acid are recycled in the same reactor system where the treatment of the biomass prior to a hydrolysis reaction occurs, thereby avoiding subsequent processing steps of the components, such as removal from the pretreatment reactor, recovery, and recycling. This treatment with acid can sometimes be referred to as "pretreatment." In one embodiment, the reactor system comprises one reaction vessel. In another embodiment, the reactor system comprises at least two reaction vessels. In yet another embodiment, the reactor system comprises at least three reaction vessels.

One exemplary embodiment of the reversible acid is α-hydroxysulfonic acid (or alpha-hydroxysulfonic acid), which can be generated as disclosed in U.S. Publication No. US 2012/0122152, entitled Treating Biomass to Produce Materials Useful for Biofuels, and filed on November 3, 2011, the disclosure of which is incorporated herein in its entirety.

For instance, α-hydroxysulfonic acid is effective for treatment of biomass hydrolyzing the biomass to fermentable sugars like pentose, such as xylose, at lower temperature while producing little to no furfural in the process. Non-limiting examples of α-hydroxysulfonic acid includes α-hydroxymethane sulfonic acid or α-hydroxyethane sulfonic acid. In particular, α-hydroxymethane sulfonic acid or α-hydroxyethane sulfonic acid can hydrolyze biomass to fermentable sugars at about 100°C. The α-hydroxysulfonic acid is revertible to its components, which are removable and recyclable as well as preferably non-acidic, unlike mineral acids such as sulfuric, phosphoric, or hydrochloric acid. The lower temperatures and pressures employed in the biomass treatment leads to lower equipment cost. Biomass pretreated in this manner prior to a hydrolysis reaction has been shown to be highly susceptible to additional saccharification, especially enzyme mediated saccharification, as compared to biomass that has not been pretreated.

The alpha-hydroxysulfonic acids have the general formula 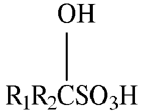 where R₁ and R₂ are individually hydrogen or hydrocarbyl with up to about 9 carbon atoms that may or may not contain oxygen can be used in the treatment of the instant invention. The alpha-hydroxysulfonic acid used in embodiments of the present invention can be a mixture of the aforementioned acids. The alpha-hydroxysulfonic acid can generally be prepared by reacting at least one carbonyl compound or precursor of carbonyl compound (e.g., trioxane and paraformaldehyde) with sulfur dioxide or precursor of sulfur dioxide (e.g., sulfur and oxidant, or sulfur trioxide and reducing agent) and water according to the following general equation 1. 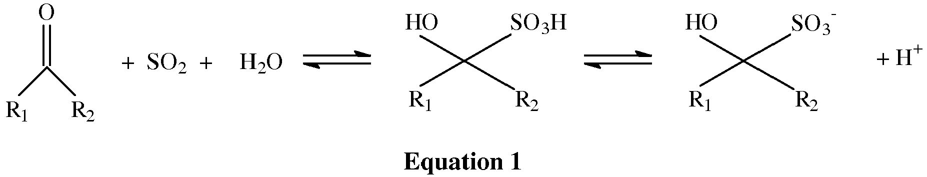 where R₁ and R₂ are individually hydrogen or hydrocarbyl with up to about 9 carbon atoms or a mixture thereof.

One example of the preparation of α-hydroxysulfonic acids is by the combination of an organic carbonyl compounds, SO₂ and water is a general reaction and is illustrated in equation 2 for acetone.

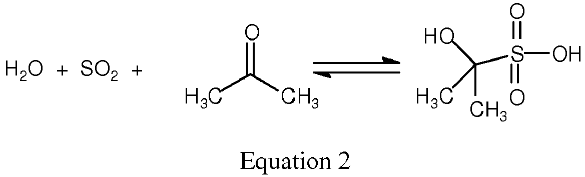

The reversible acid used in embodiments of the invention is preferably about as strong as mineral acids. For example, in one embodiment, the reversible acid used is alpha-hydroxysulfonic acid shown in Equation 2. The alpha-hydroxysulfonic acid is at least as strong as HCl since an aqueous solution ofthe adduct has been reported to react with NaCI freeing the weaker acid, HCI (see U.S. Pat. No. 3,549,319).

The reactions in equations 1 and 2 are a true equilibrium, which result in facile reversibility of the acid. That is, when heated, the equilibrium shifts towards the starting carbonyl, sulfur dioxide, and water. If the volatile components (e.g. sulfur dioxide) are allowed to depart the reaction mixture via vaporization or other methods, the acid reaction completely reverses and the solution becomes effectively neutral. Thus, by increasing the temperature and/or lowering the pressure, the sulfur dioxide can be driven off and the reaction completely reverses due to Le Châtelier's principle, the fate of the carbonyl compound is dependent upon the nature of the material employed. In various embodiments, the carbonyl is also volatile (e.g. acetaldehyde), which allows it to be recycled for subsequent reformation into the reversible acid in the vapor phase, according to embodiments of the invention. Therefore, the formation of these acids is reversible under certain conditions, such as an increased in temperature. For alpha-hydroxysulfonic acid, it reverts to its components, such as sulfur dioxide and/or aldehyde and/or ketone, which can be flashed from the mixture and condensed or absorbed elsewhere, in the same reactor or multiple reactors coupled with one another, for subsequent uses (recycling) as desired, according to embodiments of the invention.

The reversible acid used in embodiments of the invention produces very few of the undesired byproducts, such as furfurals, which are produced by other conventional mineral acids, such as sulfuric, phosphoric, or hydrochloric acid. Additionally, since the reversible acid is effectively removed from the reaction mixture following treatment, neutralization with base and the formation of salts to complicate downstream processing is substantially avoided. The ability to reverse and recycle the reversible acids also allows the use of higher concentrations than would otherwise be economically or environmentally practical. As a direct result, the temperature employed in biomass treatment can be reduced to diminish the formation of byproducts such as furfural or hydroxymethylfurfural.

It has been found that the position of the equilibrium given in equation 1 at any given temperature and pressure is highly influenced by the nature of the carbonyl compound employed, steric and electronic effects having a strong influence on the thermal stability of the acid. More steric bulk around the carbonyl tending to favor a lower thermal stability of the acid form. Thus, one can tune the strength of the acid and the temperature of facile decomposition by the selection of the appropriate carbonyl compound.

Various factors affect the conversion of the biomass feedstock in the hydrolysis reaction. In an embodiment using carbonyl or incipient carbonyl compounds (such as trioxane) with sulfur dioxide and water, these components should be added in an amount and under conditions effective to form alpha-hydroxysulfonic acids. The temperature and pressure of the hydrolysis reaction should be in the range to form alpha-hydroxysulfonic acids and to hydrolyze the biomass into fermentable sugars. The amount of carbonyl compound or its precursor and sulfur dioxide should be sufficient to produce alpha-hydroxysulfonic acids in the range from about 1 wt% to about 55 wt%, preferably from about 5 wt% to about 55 wt%, more preferably from about 10 wt% to about 55 wt%, more preferably still from about 1 wt%, about 5 wt%, or about 10 wt% to about 50 wt%, more preferably still from about 1 wt%, about 5 wt%, or about 5 wt% to about 40 wt%, based on the total solution. In certain embodiments, excess sulfur dioxide is not necessary, but any excess sulfur dioxide may be used to drive the equilibrium in equation 1 to favor the acid form at elevated temperatures.

In a preferred embodiment, the condition under which contact between the reversible acid and biomass occurs takes place at a temperature preferably at least from about 50 °C depending on the acid, such as alpha-hydroxysulfonic acid, used; although such temperature may be as low as room temperature depending on the acid and the pressure used. The contacting temperature of the hydrolysis reaction may range preferably up to and including about 150 °C depending on the acid used, such as a particular alpha-hydroxysulfonic acid. In a more preferred condition, a temperature of the reaction zone is at least from about 80°C, more preferably at least about 100°C. In a more preferred condition, a temperature of the reaction zone is in a range up to and including about 90°C to about 120 °C The reaction is preferably conducted in a reaction zone with as low a pressure as possible, if excess sulfur dioxide is used.

In one particular embodiment, the reaction is conducted at a pressure as low as about 1 barg, preferably about 4 barg, to a pressure as high as up to about 10 barg. The term "barg" refers to bar gauge as understood by one of ordinary skill in the art, and 1 bar equals to 0.1 MegaPascal. The temperature and pressure of a reaction can be optimally utilized depending on one or more factors, such as the particular alpha-hydroxysulfonic acid chosen, economic considerations of metallurgy, and containment vessels, which are known by those skilled in the art.

The amount of acid solution to "dry weight" biomass determines the ultimate concentration of fermentable sugar obtained. Thus, as high a biomass concentration as possible is desirable in certain circumstances. This is balanced by the absorptive nature of biomass with mixing, transport and heat transfer becoming increasingly difficult as the relative amount of biomass solids to liquid is increased. In certain embodiments, weight percentage of biomass solids to total liquids (consistency) may be as low as about 1% or as high as about 33% depending on the apparatus chosen and the nature of the biomass. In one embodiment, the temperature of the hydrolysis reaction can be chosen so that the maximum amount of extractable carbohydrates are hydrolyzed and extracted as fermentable sugar (more preferably pentose and/or hexose) from the biomass feedstock while limiting the formation of degradation products.

In a particular embodiment of the present invention, there is provided a reactor system having a biomass input adapted to receive a biomass containing polysaccharides and a biomass output adapted to discharge the biomass material. The reactor system is adapted to accommodate flow of the biomass material from the input to the output. The reactor system is further adapted to allow contact between the biomass and a solution containing at least one acid, which is revertible to components, which are preferably non-acidic, in response to a change in temperature and/or pressure. At or near the biomass input, there is a first reaction zone. At or near the biomass output, there is a second reaction zone. The first and second reaction zones have its own reaction conditions, for example, different temperature and/or pressure. The first reaction zone is suitable to drive the components of the reversible acid in the vapor phase to combine and form the acid onto the biomass while the second reaction zone is suitable to vaporize the reversible acid into its components, thereby substantially removing the reversible acid from the biomass at or near the biomass output. At least a portion of the acid components in the vapor phase is recycled back to the first reaction zone to be combined again into the reversible acid for treating the incoming biomass.

In one embodiment, the biomass may be introduced into a reactor system at ambient temperature. In another embodiment, the biomass is pre-heated prior to being introduced into the first reaction zone, preferably to a temperature below the temperature of the vapor phase present in the reactor system. In a preferred embodiment, the conditions of the first reaction zone is suitable to drive components of the reversible acid in the vapor phase to condense onto the biomass, which results in a further increase of the temperature of the biomass. In one embodiment, the components in the vapor phase include water, sulfur dioxide (SO₂), and one or more carbonyl components. After formation of a reversible acid, the temperature of the biomass remains below the equilibrium temperature of the reversible acid at the pressure in the reactor system to maintain the reversible acid in the combined form. This allows contact between the acid and biomass, thereby providing the desired chemical conversion of the biomass. The vapor phase of the first reaction zone is preferably virtually depleted of components of the reversible acid, such as one or more carbonyl components and SO₂, as the components are in the combined reversible acid form. As such, a preferred embodiment with the first reaction zone having conditions that drive acid formation also allows for the removal or exhaust of inert gasses, if any are used, from the reactor system without significant loss of valuable acid components.

The condition of the second reaction zone is suitable to drive the reaction to form acid components in the vapor phase from the majority of the reversible acid. This can be achieved by increasing the temperature and/or decreasing the pressure to obtain a temperature above the equilibrium temperature of the reversible acid at the pressure in the reaction system. If the temperature is increased by heating, it can be performed by direct steam or hot air injection, or by means of a heat exchange surface area. Other suitable heating means known to those skilled in the art can also be used.

In a preferred embodiment, a biomass containing polysaccharides is provided to a first reaction zone where a reversible acid is formed from one or more reversible acid components in the first reaction zone. The one or more components are preferably in the vapor phase. The biomass is contacted with the reversible acid in the first reaction zone. The biomass in contact with the reversible acid is moved from the first reaction zone to the second reaction zone where the reversible acid is separated into one or more reversible acid components, which are moved to the first reaction zone for formation of at least a portion of the reversible acid.

By providing conditions that vaporize the reversible acid to its components in the second reaction zone, the biomass can be discharged from the reaction system as treated biomass that is free or essentially free of acid while the components of the reversible acid can be collected and recycled as desired. The flow of the reversible acid components in vapor phase from the second reaction zone to the first reaction zone can be facilitated by a compressor, and/or by a flow of inert gas, such as nitrogen. Embodiments of the methods and systems described herein allow for substantial recovery of the reversible acid from a treatment round, which can be for future uses, such as subsequent treatments. In one embodiment, the recovery is about 60% to about 99.9%. In a preferred embodiment, the recovery is about 80% to about 99.5%, and more preferably, from about 90% to 99%.

In one embodiment, the temperature of the first reaction zone is lower than the temperature of the second reaction zone. In another embodiment, the reversible acid is α-hydroxysulfonic acid. In another embodiment, at least one component is a volatile compound. The reversible acid in its combined form may be introduced in the reaction system as a separate liquid stream, in combination with the biomass, or as vapor phase components, or as any combination of these options.

In one embodiment, accommodation of the flow of biomass through the reactor system is achieved by any suitable mechanism known to those skilled in the art. For example, the reaction vessel may incorporate an extruder, plug flow reactor, screw reactor, continuously stirred tank reactor (CSTR), Pandia digester, moving belt reactor, L&G type reactor or rotating reactor. A reactor system can comprise one, two or more reaction vessels to achieve any number of reaction zones to treat biomass with reversible acid and recycle at least a portion of the acid according to aspects of the invention. If more than one reaction vessel are employed, the same or any combination of suitable reaction vessels, such as those mentioned above, may be coupled to each other in parallel and/or in series.

For example, in one embodiment, three CSTR reactors may be applied in series. The first CSTR receives the biomass and a vapor phase stream of recycled reversible acid components. The second CSTR receives the solid/liquid biomass and reversible acid slurry from the first CSTR. The third CSTR is heated and/or reduced in pressure to separate the reversible acid, in component form in vapor phase, from the treated biomass to obtain a treated biomass essentially free of acid, and recycle at least part of the vapor phase back to the first CSTR.

In certain embodiments, the reaction vessel is configured with temperature and pressure controls to achieve one or more temperature and/or pressure settings in the reactor. That is, the reaction vessel is adapted with controls to provide one or more reaction zones, where each reaction zone has a different reaction condition, such as pressure and/or temperature. In certain embodiments, one or more inert gases, such as nitrogen and other noble gases, can be used as transfer agent of the volatile components of the reversible acid from one reaction zone to another reaction zone.

While the invention is susceptible to various modifications and alternative forms, specific embodiments thereof are shown by way of examples herein described in detail. It should be understood, that the detailed description thereto are not intended to limit the invention to the particular form disclosed, but on the contrary, the intention is to cover all modifications, equivalents and alternatives falling within the spirit and scope of the present invention as defined by the appended claims. Aspects of the present invention will be illustrated by the following examples, which is provided for illustration only and is not to be construed as limiting the claimed invention in any way.

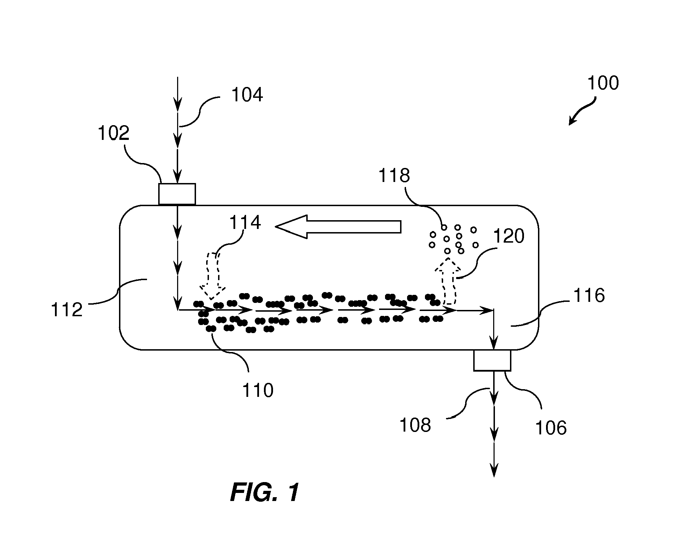

The details of specific embodiments are further provided by the descriptions of FIGS. 1, 2, 3A and 3B, which serve as illustrative purposes which are not intended to be limiting. While the Figures show exemplary reactor systems with one, two, or three reaction vessels, it is understood that other embodiments can have more than three reaction vessels to accommodate applicable operating targets. While the Figures show the reaction vessels connected in series with one another, it is understood that other embodiments can include two or more reaction vessels coupled to each other in parallel. FIG. 1 provides a particular embodiment of the invention, indicated as reactor system 100, where the reaction system comprises one reactor vessel. As shown, reactor system 100 has biomass input 102 for receiving biomass 104 and biomass output 106 for discharging treated biomass 108. Biomass 104 moves through reaction system 100 from input 102 to output 106 and exits as treated biomass 108. When biomass 104 enters reaction system 100 through input 102, it comes in contact with reversible acid 110 in first reaction zone 112, which has a temperature and pressure configured to drive the components of the reversible acid to react with one another and form the reversible acid. The formation of reversible acid 110 from components 118 are indicated by arrow 114.

Referring to FIG. 1, biomass 104 treated with reversible acid 110 moves toward output 106 where biomass 104 and reversible acid 110 pass through second reaction zone 116, which has a temperature and pressure configured to reverse the acid reaction where the reversible acid separates into its components 118. The reverse acid reaction from reversible acid 110 to components 118 are indicated by arrow 120. At least a portion of components 118 are volatile and flow toward reaction zone 112 to combine into reversible acid 110 and starts the cycle once more. In one embodiment, the movement of components 118 from second reaction zone 116 to first reaction zone 112 is further facilitated by a flow or stream of one or more inert gases, such as nitrogen and/or noble gases. If one or more inert gasses are used, reaction system 100 can further include an input stream in or near second reaction zone 116 and a purge (output) stream in or near first reaction zone 112 for the inert gasses (both not shown). Reaction system 100 can further include a liquid stream input (not shown) in or near first reaction zone 112 to allow feeding of the reversible acid in its combined form into reaction system 100. In addition to or as an alternative, reaction system 100 can include a vapor stream input (not shown) in or near second reaction zone 116 to allow feeding of acid components into reaction system 100. The introduction of the reversible acid in its combined form and/or its components can occur at the initial phase of the biomass treatment and/or any time after depending on the operating conditions and desired operating targets. In one embodiment, routing of components 118 from second reaction zone 116 to first reaction zone 112 takes place externally of the reaction vessel. In addition or as an alternative, reactor system 100 can employ a compressor (not shown) to assist with directing components 118 from second reaction zone 116 to first reaction zone 112. In such an embodiment, the routing of components 118 from second reaction zone 116 to first reaction zone 112 takes place externally of the reaction vessel. In another embodiment, the routing of components 118 from second reaction zone 116 to first reaction zone 112 takes place externally of the reaction vessel without employing the compressor.

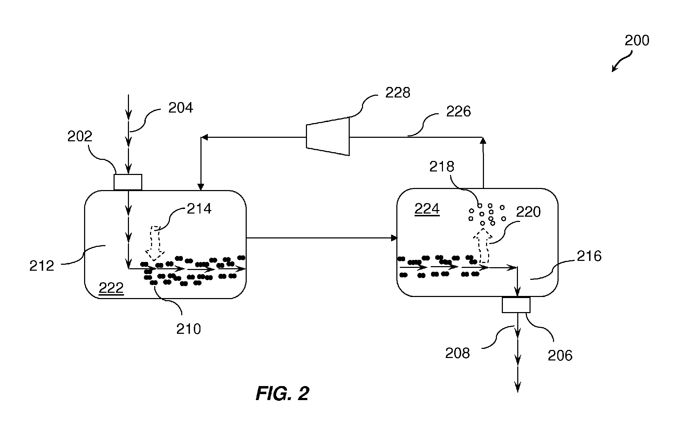

FIG. 2 provides another particular embodiment of the invention, indicated as reactor system 200, where the reaction system comprises two reactor vessels, first reactor vessel 222 and second reactor vessel 224 coupled to one another to provide and receive various materials between each other. First and second reactor vessels 222 and 224 can be the same type of vessel or different types. Each reactor vessel can be selected from any of the following: plug flow reactor, screw reactor, continuously stirred tank reactor, Pandia digester, moving belt reactor, L&G type reactor, rotating reactor, or any other suitable reactor. First reactor vessel 222 has biomass input 202 for receiving biomass 204, and second reactor vessel 224 has biomass output 206 for discharging treated biomass 208. Biomass 204 moves through reaction system 200 from first reactor vessel 222 to second reactor vessel 224 and exits as treated biomass 208. When biomass 204 enters first reaction vessel 222 through input 202, it comes in contact with reversible acid 210 in first reaction zone 212, which is in first reaction vessel 222. First reaction zone 212 has a temperature and pressure configured to drive the components of the reversible acid to react and form reversible acid 210. The formation of reversible acid 210 from non-acidic components 218 are indicated by arrow 214.

Referring to FIG. 2, biomass 204 in contact with reversible acid 210 moves from first reaction vessel 222 to second reaction vessel 224 and enters second reaction zone 216, which has a temperature and pressure configured to reverse the acid reaction where the reversible acid separates into its components 218. In one embodiment, the temperature of second reaction zone 216 is above the equilibrium temperature of reversible acid 210 at the temperature and pressure of the second reaction zone 216. The reverse acid reaction from reversible acid 210 to components 218 are indicated by arrow 220. At least a portion of components 218 are volatile and is routed first reaction vessel 222 via stream 226 to combine into reversible acid 210 and starts the cycle once more. As shown, compressor 228 is employed to facilitate the flow of components 218 from second reaction vessel 224 to first reaction vessel 222. Compressor 228, however, is optional and need not be included in other embodiments. In one embodiment, the movement of components 218 from second reaction vessel 224 to first reaction vessel 222 is further facilitated by one or more inert gases, such as nitrogen and/or noble gases. If one or more inert gasses are used, reaction system 200 can further include an input stream into second reaction vessel 224 and a purge stream out of first reaction vessel 222 for the inert gasses (both not shown). Reaction system 200 can further include a liquid stream input (not shown) in or near first reaction zone 212 to allow feeding of the reversible acid in its combined form into reaction system 200. In addition to or as an alternative, reaction system 200 can include a vapor stream input (not shown) in or near second reaction zone 216 to allow feeding of acid components into reaction system 200. In one embodiment, the temperature of second reaction vessel 224 is raised by heating, direct steam or hot inert gas injection can be employed. The biomass can be moved between reactor vessels using mechanisms known to those skilled in the art. Non-limiting examples include a pump or an auger.

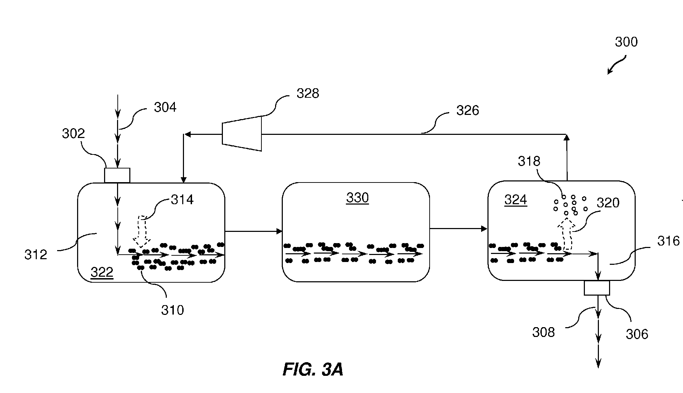

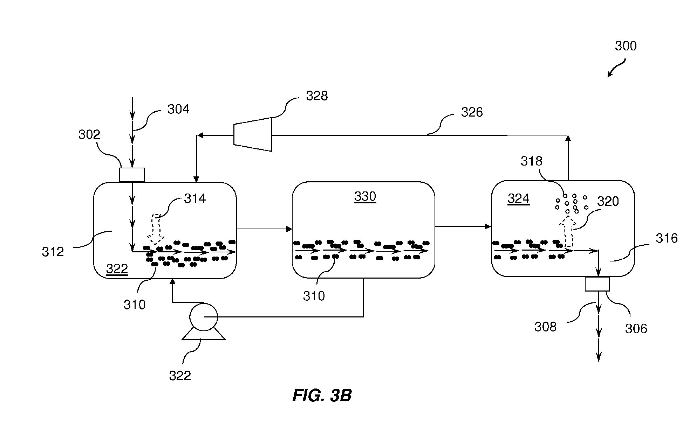

FIG. 3A provides another particular embodiment of the invention, indicated as reactor system 300, where the reaction system comprises three reactor vessels, first reactor vessel 322, second reactor vessel 324, and third reactor vessel 330. First, second, and third reactor vessels 322, 324, 330 can be the same type of vessel or any combination of two or more types. Each reactor vessel can be selected from any of the following: plug flow reactor, screw reactor, continuously stirred tank reactor, Pandia digester, moving belt reactor, L&G type reactor, rotating reactor, or any other suitable reactor. Reactor system 300 is similar to reactor system 200, except for third reactor vessel 330, which is disposed between first reactor vessel 322 and second reactor vessel 324. As shown, reactors 322, 324, and 330 are coupled to one another. Biomass 304 in contact with reversible acid 310 moves from first reactor vessel 322 to third reactor vessel 330 then to second reactor vessel 324. The time spent in each reactor is dependent on operating conditions and the desired reaction targets. In this embodiment, providing third reactor vessel 330 can serve to lengthen the treatment time, or time biomass 304 is in contact with reversible acid 310. Further, the conditions of third reactor vessel 330 can be controlled to provide a treatment environment that is more optimal than the conditions of first reactor vessel 322 where condensation and formation of reversible acid 310 occurs and/or second reactor vessel 324 where vaporization of reversible acid 310 takes place. The descriptions of FIG. 2 above, particularly similarly labeled components, are applicable to FIGS. 3A and 3B and are not repeated herein for sake of brevity. It is understood that embodiments of a reactor system according to aspects of this invention can employ more than three reactors of the same or different types and as many as desired or necessary depending on the applicable objectives. For example, additional reactor vessels can be disposed between the first and second reactors to provide sequential treatment environments that are different from one another.

FIG. 3B provides a modification to reactor system 300 to allow recycling of the reversible acid in its combined form in addition to the recycling of its components. As shown in FIG. 3B, reactor system 300 further routes at least a portion of reversible acid 310 from third reactor vessel 330 to first reactor vessel 322 through the use of pump 332. While a pump is used in this specific embodiment, it is understood that other suitable mechanisms to transfer liquid known to those skilled in art can also be employed. In a preferred embodiment, all or a portion of the biomass in third reactor vessel 330 is squeezed to remove at least a portion of reversible acid 310 in liquid form for routing back to first reactor vessel 322. If squeezing is used, it can be done by any suitable means known to those skilled in the art. One non-limiting example is a screw press. In addition or as an alternative, all or a portion of the biomass in third reactor vessel 330 can be washed to remove at least a portion of reversible acid 310. After the liquid removal, the biomass continues onto second reactor vessel 324 for vaporization of the remainder of the reversible acid and recycling of components 318 in vapor phase as described above.

The treatment reaction product, or treated biomass discharged from the reactor system according to aspects of the invention, contains fermentable sugar or monosaccharides, such as pentose and/or hexose that is suitable for further processing. The residual alpha-hydroxysulphonic acid can be removed by application of heat and/or vacuum from the fermentable sugar containing product stream to reverse the formation of alpha-hydroxysulphonic acid to its starting material to produce a stream containing fermentable sugar substantially free of the α-hydroxysulfonic acid. In particular, the product stream is substantially free of alpha-hydroxysulphonic acid, meaning no more than about 2 wt% is present in the product stream, preferably no more than about 1 wt%, more preferably no more than about 0.2 wt%, most preferably no more than about 0.1 wt% present in the product stream. The temperature and pressure will depend on the particular alpha-hydroxysulphonic acid used and minimization of temperatures employed are desirable to preserve the sugars obtained in treatment reactions.

In a preferred embodiment, a reaction mixture contains (a) a biomass containing polysaccharides, (b) a reversible acid, such as at least one α-hydroxysulfonic acid, and (c) water. When some of the biomass is treated, the reaction mixture contains (a) a biomass containing polysaccharides, (b) the reversible acid, e.g., at least one α-hydroxysulfonic acid (c) water, and (d) at least one fermentable sugar. Prior to being discharge from the reactor system, the biomass preferably contains essentially no acid.

In one embodiment, the cellulose containing product stream can further be hydrolyzed by other methods, for example by enzymes to further hydrolyze the biomass to sugar products containing pentose and hexose (e.g., glucose) and fermented to produce alcohols such as disclosed in U.S. Publication No. 2009/0061490 and U.S. Pat. No. 7,781,191, the disclosures of which are hereby incorporated by reference in their entirety.

In yet another embodiment, the fermentable sugar can be converted to furfural or hydroxymethylfurfural (HMF) or further fermented to alcohols. Although in some embodiments it may be desirable to minimize the formation of furfurals, if formation of furfurals is desired, the acid containing solution of step (b) may be further heated to a temperature in the range of from about 110°C to about 160°C, more preferably in the range of from about 120°C to about 150°C to form at least one furfural containing product stream. In one embodiment, the temperature of step (b) is maintained to a temperature of about 100°C or less if it is desirable to obtain minimal furfural in the product stream.

In yet another embodiment, the fermentable sugars can be converted to higher hydrocarbons as a biofuel component using catalytic hydrogenation and condensation techniques rather than further hydrolysis by enzyme and fermentation. The fermentable sugar containing product is preferably contacted with hydrogen in the presence of a hydrogenolysis catalyst to form a plurality of oxygenated intermediates, and then further processing the oxygenated intermediates to produce a fuel blend in one or more processing reactions. In an embodiment, a condensation reaction can be used along with other reactions to generate a fuel blend and may be catalyzed by a catalyst comprising acid or basic functional sites, or both to product a liquid fuel. As used herein, the term "higher hydrocarbons" refers to hydrocarbons having an oxygen to carbon ratio less than at least one component of the biomass feedstock. As used herein the term "hydrocarbon" refers to an organic compound comprising primarily hydrogen and carbon atoms, which is also an unsubstituted hydrocarbon. In certain embodiments, the hydrocarbons of the invention also comprise heteroatoms (e.g., oxygen or sulfur) and thus the term "hydrocarbon" may also include substituted hydrocarbons.

In one such example, the fermentable sugar containing product stream may be further processed to produce mixtures of C4+ compounds useful for biofuels such as described in U.S. Publication No. US2011/0154721 and U.S. Patent Application No. 13/106,509, filed May 12, 2011, the disclosures of which are hereby incorporated by reference in their entirety. As another such example, the fermentable sugar containing product stream may be further processed to produce mixtures of C4+ compounds useful for biofuels such as described in U.S. Publication No. 20080216391, the disclosure of which is hereby incorporated by reference in its entirety. The solid feed may also be suitable for use in fast pyrolysis reactions leading to fuels and chemicals.

The term "fermentable sugar" refers to oligosaccharides and monosaccharides that can be used as a carbon source (e.g., pentoses and hexoses) by a microorganism in a fermentation process. It is contemplated that fermentable sugar may be fermented as described above, but may also be processed by other methods without fermentation to produce fuels as described above. The term "pentose" refers to monosaccharides with five carbon atoms. The term "hexose" refers to monosaccharides with six carbon atoms.

In an enzymatic hydrolysis-fermentation processes the pH of the pretreated feedstock to the enzymatic hydrolysis is preferably adjusted so that it is within a range that is optimal for the cellulase enzymes used. Generally, the pH of the pretreated feedstock is adjusted to within a range of about 3.0 to about 7.0, or any pH there between.

The temperature of the treated feedstock is adjusted so that it is within the optimum range for the activity of the cellulase enzymes. Generally, a temperature of about 15°C to about 100°C, about 20°C to about 85°C, about 30°C to about 70°C preferably or any temperature there between, is suitable for most cellulase enzymes. The cellulase enzymes and the β-glucosidase enzyme are added to the pretreated feedstock, prior to, during, or after the adjustment of the temperature and pH of the aqueous slurry after pretreatment. Preferably, the cellulase enzymes and the β-glucosidase enzyme are added to the pretreated lignocellulosic feedstock after the adjustment of the temperature and pH of the slurry.

By the term "cellulase enzymes" or "cellulases," it is meant a mixture of enzymes that hydrolyze cellulose. The mixture may include cellobiohydrolases (CBH), glucobiohydrolases (GBH), endoglucanases (EG), and β-glucosidase. By the term "β-glucosidase", it is meant any enzyme that hydrolyzes the glucose dimer, cellobiose, to glucose. In a non-limiting example, a cellulase mixture may include EG, CBH, and β-glucosidase enzymes.

The enzymatic hydrolysis may also be carried out in the presence of one or more xylanase enzymes. Examples of xylanase enzymes that may also be used for this purpose and include, for examples, xylanase 1, 2 (Xyn1 and Xyn2) and β-xylosidase, which are preferably present in xylanases mixtures.

The process can be carried out with any type of cellulase enzymes, regardless of their source. Non-limiting examples of cellulases which may be used include those obtained from fungi of the *genera Aspergillus, Humicola,* and *Trichoderma, Myceliophthora, Chrysosporium* and from bacteria of the genera *Bacillus, Thermobifida and Thermotoga.* In some embodiments, the filamentous fungal host cell is an Acremonium, Aspergillus, Aureobasidium, Bjerkandera, Ceriporiopsis, Chrysosporium, Coprinus, Coriolus, Cryptococcus, Filibasidium, Fusarium, Humicola, Magnaporthe, Mucor, Myceliophthora, Neocallimastix, Neurospora, Paecilomyces, Penicillium, Phanerochaete, Phlebia, Piromyces, Pleurotus, Schizophyllum, Talaromyces, Thermoascus, Thielavia, Tolypocladium, Trametes, or Trichoderma cell.

The cellulase enzyme dosage is chosen to convert the cellulose of the pretreated feedstock to glucose. For example, an appropriate cellulase dosage can be about 0.1 to about 40.0 Filter Paper Unit(s) (FPU or IU) per gram of cellulose, or any amount there between. The term Filter Paper Unit(s) refers to the amount of enzyme required to liberate 2 mg of reducing sugar (e.g., glucose) from a 50 mg piece of Whatman No. 1 filter paper in 1 hour at 50° C at approximately pH 4.8.

The following experiments demonstrate particular aspects of the invention for illustrative purposes not intended to limit the scope of the invention.

### ACID COMPONENTS RECYCLING EXPERIMENTS

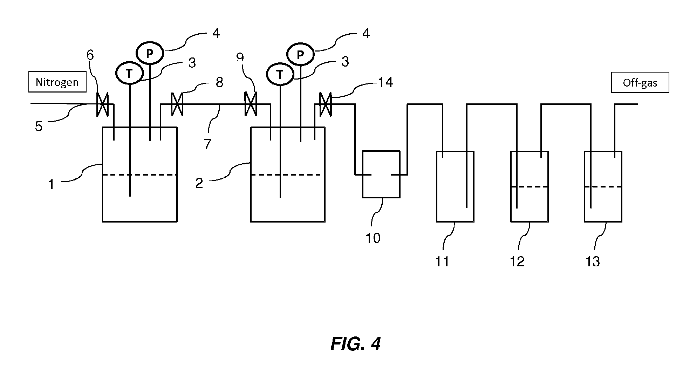

FIG. 4 provides a diagram of instrumentation configuration employed in a proof-of-concept experimental set up to show recycling of the reversible acid through condensation of the acid components after treating biomass with the reversible acid. Tables 1 - 5 below provide conditions and results from twelve experiments carried out using the instrumentation depicted in FIG. 4.

### Equipment:

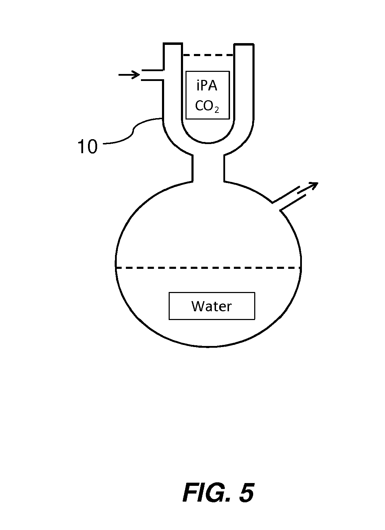

Referring to FIG. 4, reactor 1 and reactor 2 are two identical 250 ml Premex autoclaves, made of Hastelloy C22, and equipped with electrical heating (not shown), temperature detection 3 and pressure detection 4. Nitrogen is supplied to the experimental system at reactor 1 via stream 5, which can be controlled by valve 6. No stirring is applied in the set of experiments described herein. Reactor 1 and reactor 2 are in fluid communication; particularly, they are connected via tube 7, which is a ¼" stainless steel tube. Fluid flowing through tube 7 can be controlled, such as opened or closed, by two valves, valve 8 and valve 9. Each of valves 8 and 9 is located close to its respective reactor. As shown, the experimental set up further includes cold trap 10, flask 11, flask 12, and flask 13, all of which are in serial fluid communication with each other and reactors 1 and 2. Valve 14 controls flow between reactor 2 and cold trap 10, which is configured to remove condensable materials from gas flowing through it. Any cold trap known to those skilled in the art can be used. FIG. 5 shows a diagram of cold trap 10, which is one exemplary configuration of a cold trap that can be employed. As shown, cold trap 10 has a U-shaped top tube section adapted to hold material at low temperatures, such as liquid nitrogen or solid carbon dioxide (CO₂), to create a cold section to which gas flowing through cold trap 10 is exposed. For these experiments, solid carbon dioxide sitting in isopropyl alcohol (iPA) was used. The U-shaped section is in fluid communication with the bottom section which contains water to receive any condensable material in the gas that has fallen out of the gas phase after exposure to the cold section. The flow direction of the gas through cold trap 10 is indicated by the arrows in FIG. 5.

Flasks 11, 12, and 13 are wash flasks configured to remove remaining traces of SO₂ and/or acetaldehyde (if present) from gas flowing through it. As shown, flask 11 is empty to avoid back-flow. Flasks 12 and flask 13 are partly filled with 1M NaOH solution.

When nitrogen is supplied to reactor 1, it flows from reactor 1 to reactor 2, via tube 7, then through cold trap 10, which removes condensable materials from the nitrogen. After passing through cold trap 10, the nitrogen passes through flasks 11, 12, and 13 for removal of remaining traces of SO₂ and/or acetaldehyde (if present) from the nitrogen. The nitrogen is then discharged from flask 13 as off-gas.

Experiments 1 - 10 and 12 of Tables 1 - 5 were carried out using the instrumentation configuration shown in FIGS. 4 - 5. Experiment 11 was executed in the absence of cold trap 10 with reactor 2 directly connected to flask 1 and without biomass in reactor 2.

### Experimental Description:

In a typical experiment for these twelve experiments, reactor 1 is initially charged with a certain amount of hydroxyethylsulfonic acid (HESA), which is the product formed from acetaldehyde, SO₂ and water, in dilute form and a certain amount of biomass, which is milled corn stover. Reactor 2 is initially charged with a certain amount of water and milled corn stover. Table 1 provides the starting amounts of materials in each reactor for each experiment.

**Table 1.**

| | **Reactor 1** | | | | | **Reactor 2** | | | |
|---|---|---|---|---|---|---|---|---|---|
| **Exp.Nr.** | **HESA concentration** | **Dilute HESA** | **HESA** | **Corn stover** | **Corn stover** | **Water** | **Acetaldehyde** | **Corn stover** | **Corn stover** |
| **(#)** | **(%w)** | **(g)** | **(g)** | **(g wet)** | **(g dry)** | **(g)** | **(g)** | **(g wet)** | **(g dry)** |
| 1 | 10,0 | 104,41 | 10,44 | 10,43 | 9,78 | 44,50 | (-) | 8,38 | 7,85 |
| 2 | 9,5 | 88,09 | 8,35 | 8,38 | 7,85 | 34,82 | (-) | 6,64 | 6,22 |
| 3 | 7,8 | 86,10 | 6,68 | 6,64 | 6,22 | 23,45 | (-) | 5,33 | 5,00 |
| 4 | 9,2 | 58,17 | 5,35 | 5,33 | 5,00 | 12,80 | (-) | 4,29 | 4,02 |
| 5 | 10,1 | 42,37 | 4,28 | 4,29 | 4,02 | 14,64 | (-) | 3,44 | 3,22 |
| 6 | 10,0 | 100,06 | 10,01 | 10,00 | 9,37 | 50,06 | (-) | 8,00 | 7,50 |
| 7 | 9,9 | 80,06 | 7,96 | 8,00 | 7,50 | 44,00 | (-) | 6,41 | 6,01 |
| 8 | 9,5 | 66,51 | 6,31 | 6,41 | 6,01 | 30,97 | (-) | 5,11 | 4,79 |
| 9 | 10,6 | 47,15 | 5,00 | 5,11 | 4,79 | 20,56 | (-) | 4,05 | 3,80 |
| 10 | 11,4 | 34,61 | 3,94 | 4,05 | 3,80 | 10,29 | (-) | 3,03 | 2,84 |
| 11 | 5,0 | 79,87 | 3,99 | 7,98 | 7,48 | 75,81 | (-) | 0,00 | 0,00 |
| 12 | 10,0 | 80,40 | 8,04 | 8,04 | 7,54 | 57,94 | 0,86 | 6,43 | 6,03 |

The amounts of starting materials in reactors 1 and 2 are designed to provide a final liquid to biomass ratio (about 1:10) as well as the final acid strength in reactor 2 that is comparable to the initial conditions in reactor 1 (preferably about 10%w HESA). The amounts for these experiments shown in Table 1 are determined based on previous experience that about 80% of the acid is usually recovered in and approximately 30 g of liquid is usually received by reactor 2 from reactor 1 in the instrumentation configuration shown in FIG. 4. These amounts and percentages are particular to the experimental configuration used and do not limit the scope of the invention. The recovery amount is specific to this experimental configuration because small scale equipment generally tend to have lower inefficiency than larger scale equipment. Nevertheless, even at this small scale, higher efficiency can be achieved as described further below.

The first set of experiments includes Experiments 1 - 5, and the second set includes Experiments 6 - 10. In these two sets, after each experiment, reactor 2 of one experiment becomes reactor 1 of the next experiment. For example, after Experiment 1 is finished, reactors 1 and 2 were cooled, disconnected, and switched so that reactor 2 becomes reactor 1 in Experiment 2. As such, the corn stover and acid in reactor 2 of Experiment 1 becomes the starting material in Experiment 2. The treated biomass of reactor 1 of Experiment 1 is removed for enzymatic hydrolysis. Fresh corn stover and water is placed into reactor 2 of Experiment 2 in the amount indicated above, and Experiment 2 can be implemented. The switching is then repeated. The amount of acid for each set of experiments comes from what was initially added in the first acid. That is, more acid is not being added to each experiment.

After finalizing Experiment 6, 19.9 g of water was added to reactor 2 to adjust the HESA concentration to about 10%w, and a sample of 0.5 g liquid was taken for NMR analysis, resulting in 80.06 g Dilute HESA in Reactor 1 at the start of Experiment 7.

Initially, reactors 1 and 2 and valves 6, 8, 9, and 14 are closed. Reactor 1 serves as a reaction zone for treatment of the corn stover with the HESA, and it is heated to a certain temperature for 60 min. Reactor 2 starts out at ambient temperature, which may be about 20° C to about 25° C. After this heating time, valves 8 and 9 are opened and remain open for about 5 minutes, allowing at least a portion of the vapor phase of reactor 1 to flow to reactor 2 to equalize the pressure between reactors 1 and 2. After equalization, valve 14 is opened to allow at least a portion of the vapor phase of reactors 1 and 2 to flow through cold trap 10 and flasks 11, 12, and 13 and exit the system as off gas, thereby releasing the pressure of reactors 1 and 2 to ambient pressure. Meanwhile, reactor 1 continues to be heated during the opening of valves 8, 9, and 14.

After the pressure has been released, valves 8 and 14 are closed, and valve 6 is opened to let the nitrogen enter reactor 1, and it is closed after the pressure reaches about 3.2 barg. After the pressure of reactor 1 has reached 3.2 barg, valve 8 is opened to allow the pressure in reactors 1 and 2 equalize to 1.6 barg each. Valve 14 is partially opened to allow the pressure in the ractors to dissipate. Valve 14 is closed and valve 6 opened and closed again once the pressure in reactors 1 and 2 have reached 3.2 barg. Valve 14 is again partially opened to allow the pressure in the reactos to slowly dissipate. Table 2 provides the temperature set for reactor 1 during pretreatment and the corresponding temperature of reactor 2 during the flashing period, which is when the nitrogen is purged from the system. As indicated in Table 2, the temperature of reactor 2 generally increases. The temperature of reactor 2 during flashing was not determined in Experiments 11 and 12.

**Table 2**

| | **Reactor 1** | | **Reactor 2** |
|---|---|---|---|
| **Exp.Nr.** | **Temperature** | **Time** | **Temperature** |
| **(#)** | **(°C)** | **(min)** | **(°C)** |
| 1 | 120 | 60 | 46,0 |
| 2 | 120 | 60 | 30,4 |
| 3 | 120 | 60 | 57,7 |
| 4 | 120 | 60 | 56,8 |
| 5 | 120 | 60 | 30,7 |
| 6 | 100 | 60 | 30,7 |
| 7 | 100 | 60 | 54,2 |
| 8 | 100 | 60 | 24,9 |
| 9 | 100 | 60 | 33,5 |
| 10 | 100 | 60 | 32,1 |
| 11 | 120 | 60 | n.d. |
| 12 | 100 | 60 | n.d. |

After the nitrogen is purged, the heating of reactor 1 is stopped, and reactors 1 and 2 are opened. The content of reactor 1 is removed and subjected to pH measurement and filtered. The solid fraction reactor 1 is washed with approximately 450 ml water for subsequent enzymatic hydrolysis. A sample of about 0.5ml is removed from reactor 2 to determine the HESA concentration by proton nuclear magnetic resonance. After which, as described above, for Experiments 1 - 5 and Experiments 6 - 10, reactor 2 replaces reactor 1 in the subsequent experiment. Table 3 provides the results for the treated biomass of reactor 1, including mass, percent liquefaction, final pH, final pH after a small amount of water is added to measure pH, and % of HESA remaining on the treated biomass. Table 3 also provides the amount of liquid and acid in reactor 2 for each experiment.

**Table 3**

| | **Reactor 1** | **Reactor 1** | **Reactor 1** | **Reactor 1** | **Reactor 1** | **Reactor 2** | **Reactor 2** |
|---|---|---|---|---|---|---|---|
| **Exp.Nr.** | **Solid residue** | **liquefaction** | **Final pH** | **Final pH (+H2O)** | **HESA, going w residue** | **Total liquid** | **HESA** |
| **(#)** | **(g dry)** | **(%w)** | **(-)** | **(-)** | **(%w on initial HESA)** | **(g)** | **(g)** |
| 1 | 5,52 | 43,5 | 1,36 | 1,53 | 3,36 | 88,54 | 8,35 |
| 2 | 4,63 | 41,1 | 1,34 | 1,48 | 2,74 | 86,10 | 6,68 |
| 3 | 3,52 | 43,4 | 1,46 | 1,46 | 3,27 | 58,17 | 5,35 |
| 4 | 2,83 | 43,4 | 1,26 | 1,39 | 4,02 | 42,37 | 4,28 |
| 5 | 2,31 | 42,7 | 1,14 | 1,40 | 4,24 | 38,21 | 3,42 |
| 6 | 6,29 | 32,9 | 1,35 | 1,35 | 4,96 | 60,66 | 8,00 |
| 7 | 5,02 | 33,1 | 2,77 | 2,97 | 0,15 | 67,02 | 6,36 |
| 8 | 3,77 | 37,2 | 2,09 | 2,23 | 0,79 | 47,65 | 5,05 |
| 9 | 3,06 | 36,2 | 1,75 | 1,97 | 1,43 | 35,11 | 4,00 |
| 10 | 2,27 | 40,2 | 2,36 | 2,75 | 0,30 | 22,87 | 3,15 |
| 11 | 4,55 | 39,2 | n.d. | 3,26 | 0,09 | n.d. | n.d. |
| 12 | 4,96 | 34,2 | 2,20 | 2,28 | 0,58 | 81,79 | n.d. |

In general, a higher percent of liquefaction indicates a better acid treatment because the higher percent indicates a higher biomass percentage had been dissolved during treatment. As Table 3 indicates, a substantial amount of acid is removed from and does not remain with the treated biomass. For example, the highest percentage remaining on the treated biomass was about 5%, and the lowest was about 0.09%, which essentially removed all HESA from the treated biomass. The trend of the results shown in Table 4 indicates that there is more acid loss going with the biomass when the treatment temperature is higher. For example, the losses for Experiments 1 - 5, which were conducted at 120 degrees C, ranged between about 3% - about 4% while the losses for Experiments 6 - 10, which were conducted at 100 degrees C, were more in the range of about 0.5% - about 1.5%, except for the one outlier being Experiment 6.

Cold trap 10 and flasks 11, 12, and 13, are weighed after each experiment to determine the amount of components of the reversible acid, if any, are recovered from the vapor phase of reactors 1 and 2, as well as during the nitrogen purging. Table 4 provides the mass balance of cold trap 10 and flasks 11, 12, and 13 after each experiment and the HESA recovered per pass.

**Table 4**

| | **Reactor 1** | **Reactor 2** | **Cold trap** | **Flask 1** | **Flask 2** | **Flask 3** | **System** | **HESA recovered** |
|---|---|---|---|---|---|---|---|---|
| **Exp.Nr.** | **Weight lost** | **Weight gained** | **Weight gained** | **Weight gained** | **Weight gained** | **Weight gained** | **Loss from system** | **per pass** |
| **(#)** | **(g)** | **(g)** | **(g)** | **(g)** | **(g)** | **(g)** | **(g)** | **(%w, NMR)** |
| 1 | 43,84 | 43,51 | 0,40 | 0,01 | 0,10 | -0,03 | -0,15 | n.d. |
| 2 | 52,93 | 50,86 | 0,19 | 0,18 | -0,22 | 0,03 | 1,89 | n.d. |
| 3 | 38,39 | 34,39 | 0,26 | -0,02 | 0,11 | -0,04 | 3,69 | n.d. |
| 4 | 29,81 | 29,30 | -0,02 | -0,03 | 0,13 | 0,00 | 0,43 | n.d. |
| 5 | 24,87 | 23,35 | -0,09 | -0,02 | 0,12 | 0,01 | 1,50 | 71 |
| 6 | 12,78 | 10,10 | 0,59 | 0,03 | 0,10 | 0,00 | 1,96 | 59 |
| 7 | 24,71 | 22,62 | 0,04 | 0,02 | 0,19 | 0,00 | 1,84 | 63 |
| 8 | 18,37 | 16,36 | -0,08 | -0,02 | 0,09 | 0,00 | 2,02 | 109 |
| 9 | 16,24 | 14,30 | -0,02 | 0,00 | 0,07 | -0,04 | 1,93 | 96 |
| 10 | 13,71 | 12,39 | -0,07 | -0,04 | 0,05 | 0,00 | 1,38 | 79 |
| 11 | 30,72 | -69,60 | n.d. | 93,02 | 0,06 | -0,06 | 7,30 | n.d. |
| 12 | 23,97 | 22,59 | -0,03 | 0,00 | 0,11 | -0,02 | 1,32 | n.d. |

The experimental set up for Experiment 11 was different from what is shown in FIG. 4. In particular, Experiment 11 was executed in the absence of a cold trap, and without biomass present in Reactor 2. Further, due to an operational error, part of the liquid phase of reactor 2 was transferred to flask 11 by means of a dip tube in reactor 2 (dip tube not shown in FIG. 4).

The results in Table 4 indicates that there is significant recovery of the reversible acid from reactor 1. In particular, the average HESA recovery of Experiments 1 - 5 is about 71% per pass, and the average HESA recovery of Experiments 6 - 10 is about 81% per pass, as determined by NMR. There is substantial recovery even when the temperature is close to about 60 degrees C, such as that of Experiments 3 and 4, where the temperature of reactor 2 was at about 57.7 degrees C and 56.8 degrees C (see Table 2). Further, as indicated by at least Experiment 9, there can be a high recovery percentage, such as about 96% even in a small scale configuration. The amount of 109% recovery shown for Experiment 9 represents an experimental error as more than 100% cannot be recovered.

Table 5 shows the glucan and xylan content of the biomass of each experiment before (original) and after the acid treatment. As shown below, the after treatment the majority of the glucan is recovered in the solid state and less than 10% of the original glucan is dissolved by the treatment. Further, the majority of the xylan is converted during treatment of the biomass into xylan oligomers and xylose monomer and dissolved in the liquid phase. About 1.7%w xylan of the original corn stover basis is left after treatment at 120° C, and about 4.5%w of the original corn stover basis is left after treatment at 100° C. The results in Table 5 show that the treatment with reversible acid according to certain aspects of the invention substantially preserve the initial amount of glucose in the biomass and convert a substantial amount of xylan into more preferred forms.

**Table 5**

| **Exp.Nr.** | **Glucan** | **Xylan** | **Glucan** | **Xylan** |
|---|---|---|---|---|
| **(#)** | **(%w on residue)** | **(%w on residue)** | **(%w original)** | **(%w original)** |
| 1 | 57,3 | 2,4 | 32,4 | 1,37 |
| 2 | 57,3 | 2,8 | 33,8 | 1,67 |
| 3 | 56,7 | 3,2 | 32,1 | 1,80 |
| 4 | 56,8 | 2,8 | 32,2 | 1,60 |
| 5 | 57,8 | 3,1 | 33,1 | 1,76 |
| 6 | 49,5 | 6,6 | 33,2 | 4,45 |
| 7 | 49,9 | 7,2 | 33,4 | 4,81 |
| 8 | 50,8 | 7,3 | 31,9 | 4,56 |
| 9 | 53,5 | 7,4 | 34,1 | 4,71 |
| 10 | 53,2 | 7,2 | 31,8 | 4,29 |

### Enzymatic hydrolysis

Enzymatic hydrolysis of the washed solid from reactor 1 of each experiment is executed at about 2% cellulose assay. This was done by adding 50 ml water, buffered at pH 5 with sodium citrate, and an amount of washed pretreated corn stover corresponding to 2%w cellulose to a 250 ml Erlenmeyer, which was shaken in an oven having a temperature of 50° C. After temperature equilibration, cellulase Genencor 220 was added at 15 mg enzyme per g cellulose. Conversion from cellulose to glucose is determined at 24, 48, 72 and 168 h by measuring the glucose concentration.

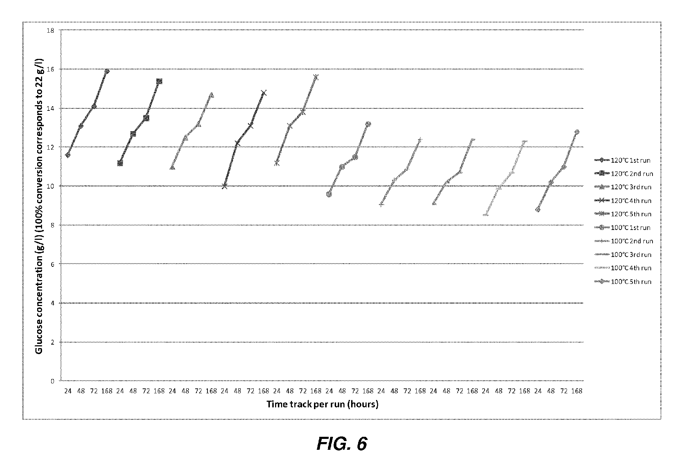

FIG. 6 shows the results for Experiments 1 - 10. Experiment 11 resulted in 8.88 g/l 10.4 g/l; 11.2 g/l and 12.2 g/l glucose concentration at 24, 48, 72, and 168 hours residence time, respectively. Enzymatic hydrolysis results of Experiment 12 are not available. FIG. 6 shows glucose concentrations that are higher than what would have been obtained without the acid treatment. Table 6 below provides the values plotted in FIG. 6.

**Table 6**

| **Exp.Nr.** | **24 hrs** | **48 hrs** | **72 hrs** | **168 hrs** |
|---|---|---|---|---|
| **(#)** | **(g/l)** | **(g/l)** | **(g/l)** | **(g/l)** |
| 1 | 11,6 | 13,1 | 14,1 | 15,9 |
| 2 | 11,2 | 12,7 | 13,5 | 15,4 |
| 3 | 11 | 12,5 | 13,2 | 14,7 |
| 4 | 9,99 | 12,2 | 13,1 | 14,8 |
| 5 | 11,2 | 13,1 | 13,8 | 15,6 |
| 6 | 9,6 | 11 | 11,5 | 13,2 |
| 7 | 9,07 | 10,3 | 10,9 | 12,4 |
| 8 | 9,09 | 10,3 | 10,7 | 12,4 |
| 9 | 8,52 | 9,87 | 10,7 | 12,2 |
| 10 | 8,81 | 10,3 | 11 | 12,8 |
| 11 | 8,88 | 10,4 | 11,2 | 12,2 |
| 12 | n.d. | n.d. | n.d. | n.d. |

In addition, Table 7 below shows that little or no furfural is produced during the process, thereby eliminating or significantly reducing the negative effects associated with other conventional pretreatment process.

**Table 7**

| **Exp.Nr.** | **Furfural** | **Furfural** |
|---|---|---|
| **(#)** | **(%w)** | **(g)** |
| 1 | | |
| 2 | | |
| 3 | | |
| 4 | | |
| 5 | 0,24 | 0,09 |
| 6 | 0,00 | 0,00 |
| 7 | 0,00 | 0,00 |
| 8 | 0,02 | 0,01 |
| 9 | 0,03 | 0,01 |
| 10 | 0,08 | 0,02 |

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as the presently preferred embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

## Claims

1. A reactor system comprising:
a first reaction zone having a first set of reaction conditions adapted to drive formation of a reversible acid from one or more components in a vapor phase of the reactor system;
a second reaction zone having a second set of reaction conditions adapted to drive separation of the reversible acid to its components;
a biomass input adapted to introduce a biomass into the first reaction zone; and
a biomass output adapted to discharge the biomass from the second reaction zone;
wherein the reactor system is adapted to move the biomass from the first reaction zone to the second reaction zone; and
wherein the reactor system is adapted to move one or more components of the reversible acid from the second reaction zone to the first reaction zone for formation of at least a portion of the reversible acid.

2. The system of claim 1 wherein the first reaction zone and the second reaction zone are in one reactor vessel.

3. The system of claim 2 wherein the reactor system is adapted to move the components from the second reaction zone to the first reaction zone in the one reactor vessel.

4. The system of claim 1 wherein the first reaction zone and the second reaction zone are in different reactor vessels.

5. The system of claim 4 comprising at least two reactor vessels connected to each other.

6. The system of claim 4 comprising at least three reactor vessels connected to each other.

7. The system of claim 4 wherein the reactor system is further adapted to move the reversible acid in liquid form from one reactor vessel to another reactor vessel.

8. A method comprising:
(a) providing a biomass containing polysaccharides to a first reaction zone having a first set of reaction conditions suitable to drive formation of a reversible acid from one or more components;
(b) treating the biomass by allowing the biomass to contact the reversible acid;
(c) moving the biomass in contact with the reversible acid from the first reaction zone to a second reaction zone having a second set of reaction conditions suitable to drive separation of the reversible acid to its components; and
(d) moving the one or more components from the second reaction zone to the first reaction zone for formation of at least a portion of the reversible acid.

9. The method of claim 8 wherein moving of the components from the second reaction zone to the first reaction zone is facilitated by a compressor.

10. The method of claim 8 wherein the first reaction zone is in one reactor vessel and the second reaction zone is in another reaction vessel.

11. The method of claim 8 wherein the second reaction zone has a higher temperature than the first reaction zone.

12. The method of claim 8 further comprising providing a flow of an inert gas from the second reaction zone to the first reaction zone to facilitate moving of the acid components from the second reaction zone to the first reaction zone.

13. A product obtainable by the method of claim 8.

14. A method comprising:
(a) providing a biomass containing polysaccharides to a first reaction zone and forming a reversible acid from one or more components in the first reaction zone;
(b) contacting the biomass with the reversible acid in the first reaction zone;
(c) moving the biomass in contact with the reversible acid from the first reaction zone to a second reaction zone and separating the reversible acid into one or more components in the second reaction zone;
(d) moving the one or more components from the second reaction zone to the first reaction zone for formation of at least a portion of the reversible acid.

15. The method of claim 14 wherein the first reaction zone and the second reaction zone are in one or more reactor vessels.
